(19) **Europäisches Patentamt**

**European Patent Office**

**Office européen des brevets**

(11) Veröffentlichungsnummer: **0 241 792 B1**

(12) **EUROPÄISCHE PATENTSCHRIFT**

(45) Veröffentlichungstag der Patentschrift: **20.05.92**

(51) Int. Cl.⁵: **A61B 17/58**

(21) Anmeldenummer: **87104638.9**

(22) Anmeldetag: **28.03.87**

(54) **Setz- und Einschlaginstrument für einen Knochennagel.**

(30) Priorität: **15.04.86 CH 1491/86**

(43) Veröffentlichungstag der Anmeldung:
**21.10.87 Patentblatt 87/43**

(45) Bekanntmachung des Hinweises auf die
Patenterteilung:
**20.05.92 Patentblatt 92/21**

(84) Benannte Vertragsstaaten:
**AT BE DE ES FR GB IT NL SE**

(56) Entgegenhaltungen:
**DE-A- 2 933 141**
**GB-A- 13 219**
**GB-A- 2 118 474**

(73) Patentinhaber: **GEBRÜDER SULZER AKTIENGE-
SELLSCHAFT**
**Zürcherstrasse 9**
**CH-8401 Winterthur(CH)**

(72) Erfinder: **Frey, Otto**
**Wallrütistrasse 56**
**CH-8400 Winterthur(CH)**
Erfinder: **Koch, Rudolf**
**Oberdorfstrasse 229**
**CH-8267 Berlingen(CH)**

## Beschreibung

Die Erfindung betrifft ein Setz- und Einschlaginstrument für einen Knochennagel mit einem zylindrischen Stamm, der sich zum distalen Ende hin verjüngt und mit einer widerhakenartigen Verzahnung versehen ist, wobei der Stamm an seinem proximalen Ende in einen konischen Kopf übergeht, in dessen Oberseite eine kegelförmige Zentriervertiefung angeordnet ist.

Knochennägel der genannten Art dienen zur Fixierung von Implantaten, z.B. von künstlichen Teiloberflächen von Gelenken (CH-PS 632 922) oder von Osteosynthese-Platten bzw. - in Verbindung mit Agraffen - von künstlichen Bändern und Sehnen. Um ihre Aufgabe optimal erfüllen zu können, ist es oftmals notwendig, dass diese Nägel unter einem schiefen Winkel in den Knochen eingeschlagen werden, wobei die Richtung des ursprünglichen "Ansatzes" des Nagels an den Knochen auch beim Eintreiben so genau wie möglich eingehalten werden muss.

Aufgabe der Erfindung ist es, für einen Knochennagel ein zugehöriges Setz- und Einschlaginstrument zu schaffen, mit dem die vorstehend genannten Forderungen erfüllt werden. Die Lösung dieser Aufgabe ist dadurch gekennzeichnet, dass das Setz- und Einschlaginstrument einen zylindrischen Stempel aufweist, der an seinem einen Ende in einen Zapfen mit mehreren stufenartigen Absätzen übergeht, wobei der Zapfen seinerseits in einem Zentrierkegel ausläuft, der das positive Gegenstück zu der Zentriervertiefung an der Oberseite des Nagelkopfes bildet, und dass eine mit ihrem Mittelteil auf einem Führungsteil mittleren Durchmessers des Zapfens auf- und im Gleit/Pass-Sitz verschiebbare Hülse vorgesehen ist, die beidseits ihres Mittelteils, durch Schlitze unterteilt, aufspreizbar ist, wobei der dem Stempel zugewandte Endteil, an einer konischen Einschnürung des Zapfens anliegend, in denjenigen der Absätze eingreift, der am Basisende der Einschnürung vorhanden ist, während der andere Endteil der Hülse den Kopf des Knochennagels am Zentrierkegel des Zapfens festhalten und zentrieren kann.

In Verbindung mit der kegelförmigen Zentriervertiefung des Nagels gewährleistet der Zentrierkegel des Instrumentes eine starre, koaxiale Verbindung beider Elemente, wobei die Hülse den Nagel mit ihrem einen Endteil am konischen Kopf praktisch auf dem ganzen Umfang umgreift und so ein "Auswandern" des Nagels aus der mit dem Instrument koaxialen Position beim Einschlagen verhindert.

Darüberhinaus verursacht die Hülse, die vorteilhafterweise an ihrer nagelseitigen Stirnfläche als Aufsetzfläche mit abgerundeten Kanten ausgebildet ist, am Ende des Einschlagvorganges ein selbsttätiges Lösen des Nagels aus der starren Verbindung und ein automatisches Rückstellen der Hülse in die Ausgangsposition. Dabei bewirkt die Aufspreizbarkeit des nagelseitigen Endteils der Hülse das Lösen der Verbindung zwischen dem Instrument und dem Kopf des eingeschlagenen Nagels, während die zwangsläufige Rückstellung der gelösten Hülse in die Ausgangsstellung auf dem Zapfen des Stempels durch ihren elastisch verformten und aufgespreizten stempelseitigen Endteil erfolgt.

Dabei ist es zweckmässig, wenn der konischen Einschnürung zum Zentrierkegel hin ein zylindrischer Teil mit einer Schulter vorgelagert ist, dessen Durchmesser grösser ist als derjenige des die Hülse tragenden Führungsteils; die Schulter wirkt dabei beim Einschlagen des Nagels als Anschlag für die Hülse und verhindert ein Ueberspreizen von deren stempelseitigem Endteil.

Weiterhin ist es für das Aufspreizen des unteren Hülsenendteils beim Einsetzen des Nagels vorteilhaft, wenn der Rand des konischen Kopfes nach aussen abgeschrägt ist.

Durch die konische Form des Nagelkopfes ist es weiterhin möglich, diesen Kopf mit einem Ausziehinstrument, z.B. einer Zange, zu untergreifen, wodurch ein Ausziehen des Nagels erheblich erleichtert und vereinfacht wird.

Im folgenden wird die Erfindung anhand eines Ausführungsbeispiels im Zusammenhang mit der Zeichnung näher erläutert.

Fig. 1      ist, teilweise im Schnitt, eine Ansicht eines passenden Knochennagels;

Fig. 2      zeigt das Setz- und Einschlaginstrument mit einem angesetzten Nagel nach Fig. 1;

Fig. 3 bzw. 4      stellen, gegenüber Fig. 2 vergrössert, den Zapfen bzw. die Hülse am Ende des Einschlaginstrumentes dar;

Fig. 5      gibt einen Längsschnitt V-V von Fig. 2 wieder, in dem ein an dem Einschlaginstrument fixierter Nagel vor dem Einschlagen gezeigt ist;

Fig. 6      zeigt in gleicher Darstellung wie Fig. 5 das Lösen des Instrumentes von einem eingeschlagenen Nagel;

Fig. 7      illustriert den Angriff einer Ausziehzange an einem zum Setz- und Einschlaginstrument passenden Nagel;

Fig. 8      schliesslich zeigt als Anwendungsbeispiel für das Setz- und Einschlaginstrument die Verankerung eines künstlichen Bandes mit Knochennägeln an einem Knochen.

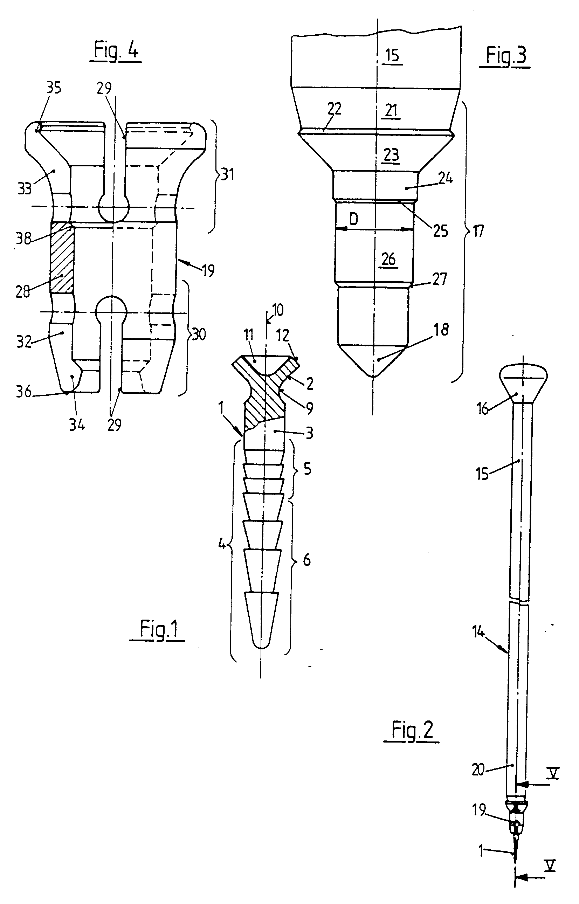

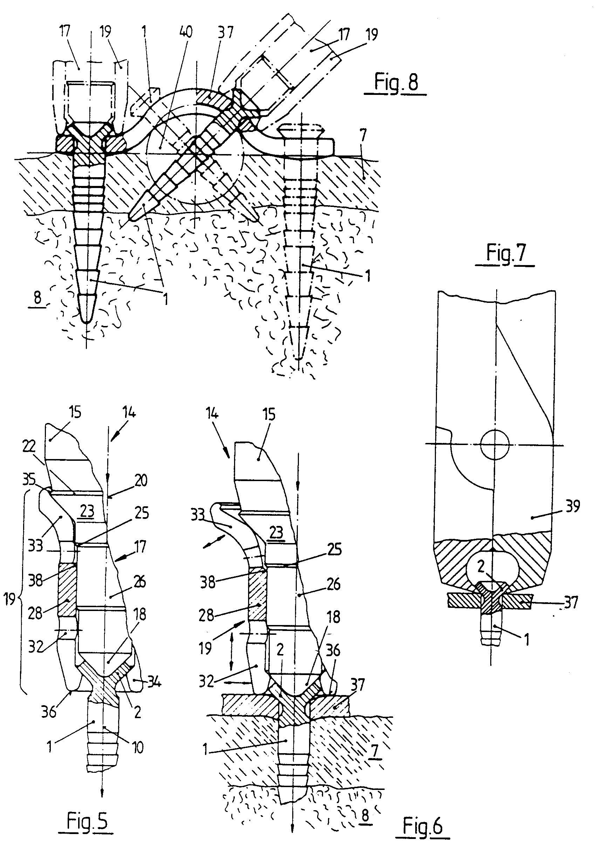

Die Grundform des Knochennagels 1 (Fig. 1) ist nahe dem Kopf 2 ein zylindrischer Stamm 3; an den Stamm 3 schliesst sich zum freien Ende hin ein konischer Bereich 4 an. Der sich verjüngende Teil 4 trägt zunächst eine relative feine Verzahnung 5, die beim Einschlagen in einen Knochen in das kortikale Gewebe 7 (Fig. 8) eindringt und mit ihren feinen Zähnen in dieses Gewebe widerhakenartig eingreift, so dass ein Herauswandern aus dem Gewebe entgegen der Einschlagrichtung sicher verhindert wird. Die Feinverzahnung 5 setzt sich fort in einer groben Verzahnung 6; diese dient zur Verankerung des Nagels 1 im spongiosen Gewebe 8 (Fig. 8).

Der Kopf 2, der über eine Einschnürung 9 mit dem Stamm 3 verbunden ist, ist konisch ausgebildet und weist auf seiner Oberseite eine mit der Nagelachse 10 konzentrische kegelförmige Zentriervertiefung 11 auf. Sein Rand 12 ist nach aussen abgeschrägt, wodurch, wie erwähnt, ein Einsetzen des Nagels 1 in eine Hülse 19 (Fig. 2) eines Setz-und Einschlaginstrumentes 14 erleichtert wird.

Das Setz- und Einschlaginstrument wird im wesentlichen von einem zylindrischen Stempel 15 gebildet, an dessen einem Ende ein knaufartiger Kopf 16 vorgesehen ist; dieser Knauf 16 dient einerseits als Handgriff für die Führung des Instrumentes durch den Operateur während des Einschlagens und andererseits als "Amboss" für ein nicht gezeigtes Schlagwerkzeug. An seinem anderen Ende geht der Stempel in einen Zapfen 17 (Fig. 3) über, dessen Durchmesser sich in mehreren Stufen zu der Basis eines Zentrierkegels 18 verringert. Dieser bildet das positive Gegenstück zu der Zentriervertiefung 11 an der Oberseite des Nagelkopfes 2.

Auf dem Zapfen 17 sitzt verschiebbar die Hülse 19, durch die der Nagel 1 am Instrument 14 so gehalten wird, dass die Instrumentenachse 20 (Fig. 2) und die Nagelachse 10 miteinander fluchten.

Für den Uebergang des Stempels 15 in den Zentrierkegel 18 ist an den Stempfel 15 zunächst ein erster Konus 21 angesetzt, der nach einer gewissen Länge von einem Absatz 22 stufenförmig abgeschlossen ist; an diesem Absatz 22 beginnt eine zweite konische Uebergangsfläche oder Einschnürung 23 des Zapfens 17, die in einen zylindrischen Teil 24 übergeht und in einer Schulter 25 endet. Es schliesst ein zylindrischer Führungsteil 26 an, an dessen Ende sich der Durchmesser des Zapfens 17 über einen weiteren Absatz 27 zum Basisdurchmesser des Zentrierkegels 18 verringert.

Der Führungsteil 26 dient zur Führung der Hülse 19, die daher mit ihrem Mittelteil 28 (Fig. 4) an den Durchmesser D des Führungsteils 26 so angepasst ist, dass sie im Gleit-/Pass-Sitz auf dem Führungsteil 26 verschiebbar ist, wie ein Vergleich der Figuren 5 und 6 verdeutlicht.

Die beiden Endteile 30 und 31 der Hülse 19 sind durch Schlitze 29 in je vier aufspreizbare Lappen 32 und 33 unterteilt, die in nasenartigen Vorsprüngen 34 und 35 enden. Die stempelseitigen Lappen 33, vor denen vom Durchmesser des Mittelteils 28 her eine den Innendurchmesser vergrössernde Stufe 38 angeordnet ist, erweitern sich zu einem Hohlkonus, der in der den Nagel 1 fixierenden und zentrierenden Stellung der Hülse 19 an der Einschnürung 23 anliegt, wobei die Nasen 35 in den Absatz 22 des Zapfens 17 eingerastet sind (Fig. 5).

Der nagelseitige Endteil 30 ist eine stufenlose Fortsetzung des Mittelteils 28, die mit ihren Nasen 34 den konischen Kopf 2 des Nagels 1 hintergreift und den Nagel 1 so mit seiner Zentriervertiefung 11 gegen den Zentrierkegel 18 drückt (Fig. 5). Die Lappen 32 des nagelseitigen Endteils 30 sind auf ihrer Stirnseite abgerundet und bilden am Ende des Einschlagvorganges eine Aufsetzfläche 36, mit der die Hülse 19 auf einem Implantat 37 (Fig. 6), z.B. einer bügelartigen Agraffe (Fig. 8), oder dem Knochen aufliegt, ehe das Lösen und Rückstellen der Hülse 19 auf dem Zapfen 17 erfolgt.

Für ein Einschlagen des Nagels 1 in einen Knochen 7, 8 (Fig. 8) empfiehlt sich beispielsweise folgendes Vorgehen:

Sind - wie in Fig. 5 gezeigt - durch ein Einschieben des Nagelkopfes 2 von unten in die aufspreizbaren Lappen 32 der Hülse 19 Instrument 14 und Nagel 1 zu einer festen Einheit mit fluchtenden Achsen 10 und 20 verbunden, so wird diese Einheit zum Einschlagen des Nagels 1 in den Knochen 7, 8 (Fig. 8) in der gewünschten Einschlagrichtung - durch Pfeile in Fig. 5 angedeutet - an den Knochen angesetzt und durch Schläge mit einem nicht gezeigten Schlagwerkzeug, beispielsweise einem Hammer, auf den Knauf 16 in den Knochen eingetrieben. Sobald die Aufsetzfläche 36 der Hülse 19 an dem Implantat 37 (Fig. 6) ansteht, verschiebt sich die Hülse 19 bei weiteren Schlagen relativ zu und auf dem Zapfen 17 nach oben. Dadurch werden die Lappen 33 an der Einschnürung 23 aufgeweitet, wobei sich ihre Nasen 35 von dem Absatz 22 lösen. Ein Anstossen der Stufe 38 an der Schulter 25 verhindert ein zu weites Verschieben der Hülse 19 nach oben und damit ein Ueberspreizen der Lappen 33.

Durch die Verschiebung der Hülse 19 wird gleichzeitig ein Aufspreizen der nagelseitigen Lappen 32 bewirkt, wodurch die "Verriegelung" des Nagelkopfes 2 durch die Nasen 34 aufgehoben wird. Die Verbindung von Instrument 14 und Nagel 1 ist damit gelöst.

Beim Abheben des Instrumentes 14 von seiner Auflage auf dem Implantat 37 oder dem Knochen erfolgt aufgrund der elatischen Vorspannung in den Lappen 33 eine automatische Rückstellung der Hülse 19 in ihre "Ausgangslage", in der die Nasen 35 am Absatz 22 einrasten und das Instrument 14 zur Aufnahme eines neuen Nagels 1 bereit ist. Die bei dem geschilderten Einschlagprozess eines Nagels 1 von der Hüls 19 und ihren Lappen 32 und 33 ausgeführten Bewegungen sind in Fig. 6 durch kleine Doppelpfeile, die Einschlag- und Eintreibrichtung für den Nagel 1 durch einfach Pfeile angedeutet.

Fig. 7 verdeutlicht, wie die konische Form des Nagelkopfes 2 den Angriff eines Ausziehinstrumentes, z.B. einer Zange 39, erleichtert, falls ein in Verbindung mit einem temporären Implantat gesetzter Nagel 1 wieder aus dem Knochen entfernt werden muss.

In Fig. 8 ist eine Anwendung für das Setz- und Einschlaginstrument wiedergegeben, bei der mehrere Nägel 1 zur Verankerung eines künstlichen Bandes 40 auf einem Knochen 7, 8 mit Hilfe einer Agraffe 37 dienen. Einerseits werden dabei Nägel 1, die je nach den Gegebenheiten des Knochens unterschiedliche Längen haben können, annähernd senkrecht zur Knochenoberfläche eingetrieben, um die Agraffe 37 zu halten; andererseits können zusätzliche Nägel die Fixierung des Bandes 40 verbessern, wenn sie zum Beispiel schräg durch das Band 40 hindurch in den Knochen 7, 8 eingeschlagen werden.

Das Setz- und Einschlaginstrument besteht bevorzugt aus einem der für chirurgische Instrumente üblichen Materialien, z.B. einem "Instrumenten"-Stahl.

**Patentansprüche**

1. Setz- und Einschlaginstrument für einen Knochennagel (1) mit einem zylindrischen Stamm (3), der sich zum distalen Ende hin verjüngt und mit einer widerhakenartigen Verzahnung versehen ist, wobei der Stamm (3) an seinem proximalen Ende in einen konischen Kopf (2) übergeht, in dessen Oberseite eine kegelförmige Zentriervertiefung (11) angeordnet ist, und das Setz- und Einschlaginstrument einen zylindrischen Stempel (15) aufweist ist **dadurch gekennzeichnet,** dass, der Stempel (15) an seinem einen Ende in einen Zapfen (17) mit mehreren stufenartigen Absätzen (22, 25, 27) übergeht, wobei der Zapfen seinerseits in einem Zentrierkegel (18) ausläuft, der das positive Gegenstück zu der Zentriervertiefung (11) an der Oberseite des Nagelkopfes (2) bildet, und dass eine mit ihrem Mittelteil (28) auf einem Führungsteil (26) mittleren Durchmessers (D) des Zapfens (17) auf- und im Gleit/Pass-Sitz verschiebbare Hülse (19) vorgesehen ist, die beidseits ihres Mittelteils (28), durch Schlitze (29) unterteilt, aufspreizbar ist, wobei der dem Stempel (15) zugewandte Endteil (31), an einer konischen Einschnürung (23) des Zapfens (17) anliegend, in denjenigen der Absätze (22) eingreift, der am Basisende der Einschnürung (23) vorhanden ist, während der andere Endteil (30) der Hülse (19) den Kopf (2) des Knochennagels (1) am Zentrierkegel (18) des Zapfens (17) festhalten und zentrieren kann.

2. Instrument nach Anspruch 1, dadurch gekennzeichnet, dass die nagelseitige Stirnfläche der Hülse (19) als Aufsetzfläche (36) mit abgerundeten Kanten ausgebildet ist.

3. Instrument nach Anspruch 1, dadurch gekennzeichnet, dass der konischen Einschnürung (23) zum Zentrierkegel (18) hin ein zylindrischer Teil (24) mit einer Schulter (25) vorgelagert ist, deren Durchmesser grösser ist als derjenige des die Hülse (19) tragenden Führungsteils (26).

**Claims**

1. An introducer for a bone nail (1), the introducer having a cylindrical stem (3) which narrows towards the distal end and has barbed toothing, the stem (3) merging at its proximal end into a conical head (2) formed on its top surface with a conical centring recess (11), the introducer having a cylindrical punch or the like (15), characterised in that the punch (15) merges at one end into a pin (17) having a number of steps or shoulders or the like (22, 25, 27), the pin (17) running out into a centring cone (18) serving as the companion element to the centring recess (11) in the top of the nail head (2), and a sleeve (19) is provided which by way of its central part (28) is movable on a guide part (26) of the central diameter (D) of the pin (17) upwards and in a snug sliding fit, the sleeve (19) being expandable on both sides of its central part (28), the same being subdivided by slots (29), the end part (31) which is near the punch (15) and which contacts a conical reduced part (23) of the pin (17) engaging those of the steps or the like (22) present at the base end of the reduced part (23), while the other end part (30) of the sleeve (19) can retain and centre the bone nail head (2) on the centring cone (18) of the pin (17).

**2.** An instrument according to claim 1, characterised in that the sleeve end face near the nail is in the form of a contact surface (36) having rounded edges.

**3.** An instrument according to claim 1, characterised in that the conical reduced part (23) is preceded in the direction towards the centring zone (18) by a cylindrical part (24), the same having a shoulder whose diameter is greater than the diamter of the guide part (26) carrying the sleeve (19).

**Revendications**

**1.** Instrument pour positionner et enfoncer un clou pour os (1) comportant une tige (3) cylindrique qui se rétrécit vers l'extrémité distale et qui est pourvue d'une denture barbelée, la tige (3) se prolongeant, à son extrémité proximale, par une tête conique (2) dans la face supérieure de laquelle est placé un creux de centrage (11) conique, l'instrument pour positionner et enfoncer comporte un poinçon (15) cylindrique et est caractérisé en ce que le poinçon (15) se prolonge, à l'une de ses extrémités, par un pivot (17) présentant plusieurs épaulements (22, 25, 27) en gradins, le pivot aboutissant de son côté à un cône de centrage (18) qui forme la pièce complémentaire positive par rapport au creux de centrage (11), sur la face supérieure de la tête de clou (2), et en ce qu'il est prévu une douille (19) qui, par sa partie centrale (28), puisse s'engager et coulisser avec un ajustement glissant/serré, sur une partie de guidage (26) de diamètre (D) moyen du pivot (17), laquelle douille, partagée par des fentes (29), peut être écartée des deux côtés de sa partie centrale (28), la partie terminale (31), tournée vers le poinçon (15), s'appliquant contre un étranglement (23) conique du pivot (17), s'engage dans celui des épaulements (22) qui se trouve sur l'extrémité de base de l'étranglement (23), tandis que l'autre partie terminale (30) de la douille (19) peut maintenir et centrer la tête (2) du clou pour os (1), sur le cône de centrage (18) du pivot (17).

**2.** Instrument selon la revendication 1, caractérisé en ce que la face frontale côté clou de la douille (19) est conçue comme une surface d'appui (36) avec bords arrondis.

**3.** Instrument selon la revendication 1, caractérisé en ce qu'il est placé devant l'étranglement conique (23), vers le cône de centrage (18), une partie cylindrique (24) avec un épaulement (25) dont le diamètre est supérieur à celui de la partie de guidage (26) portant la douille (19).

Fig. 4

Fig. 3

Fig. 1

Fig. 2

Fig. 8

Fig. 7

Fig. 5

Fig. 6